# EUROPEAN PATENT APPLICATION

(11) **EP 4 393 939 A1**
(43) Date of publication of application: **03.07.2024**
(21) Application number: 22887593.6
(22) Date of filing: 26.10.2022
(51) Int. Cl.: C07K 14/34, C12N 15/77, C12P 13/10

(54) **LYSE VARIANT AND METHOD FOR PRODUCING L-ARGININE BY USING SAME**

(30) Priority: 26.10.2021 KR 20210143660
(71) Applicant: CJ CheilJedang Corporation, Seoul 04560 (KR)
(72) Inventor: KIM, Hyo Kyung, Seoul 04560 (KR); LEE, Zeewon, Seoul 04560 (KR); CHOI, Sun Hyoung, Seoul 04560 (KR)
(74) Representative: Regimbeau
(86) International application number: PCT/KR2022/016438
(87) International publication number: WO 2023/075397

(57) **Abstract**

The present disclosure provides a LysE variant.

## Description

### [Technical Field]

The present disclosure relates to a LysE variant, a microorganism of the genus *Corynebacterium* including the same, and a method of producing L-arginine using the same.

### [Background Art]

L-arginine is used for medicinal purposes such as liver function promoters, brain function promoters, complex amino acid preparations, etc., and is a substance that has recently received attention as food such as fish cake additives, health drink additives, salt substitutes for patients with high blood pressure, etc.

Studies have been continued to use microorganisms for producing high concentrations of industrially applicable L-arginine, and there have been reports about a method of using strain variants derived from microorganisms of the genus *Brevibacterium* or *Corynebacterium,* which are glutamate-producing strains, a method of using an amino acid-producing strain of which growth is improved through cell fusion, etc.

Meanwhile, although it has been reported that lysE of a microorganism of the genus *Corynebacterium,* which is a lysine exporter, also exports L-arginine (Bellmann A, et al, Microbiology, 147:1765-1774, 2001), research is required to further increase L-arginine production.

### [Disclosure]

### [Technical Problem]

The present disclosure provides a LysE variant.

### [Technical Solution]

An object of the present disclosure is to provide a LysE variant, in which any one or more amino acids of amino acids corresponding to positions 50, 153, and 215 of SEQ ID NO: 1 are substituted with another amino acid.

Another object of the present disclosure is to provide a polynucleotide encoding the LysE variant of the present disclosure.

Still another object of the present disclosure is to provide a microorganism including the LysE variant of the present disclosure or the polynucleotide encoding the same.

Still another object of the present disclosure is to provide a method of producing L-arginine, the method including the step of culturing the microorganism of the present disclosure.

### [Advantageous Effects]

The L-arginine production yield of microorganisms may be increased by using the variant of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a LysE variant, in which any one or more amino acids of amino acids corresponding to positions 50, 153, and 215 of SEQ ID NO: 1 are substituted with another amino acid.

As used herein, the term the term "LysE" refers to a lysine exporter protein encoded by lysE gene.

Specifically, the LysE may be a naturally occurring polypeptide or a wild-type polypeptide, may be a mature polypeptide thereof, and may include a variant thereof or a functional fragment thereof, but any one may be included without limitation as long as it may be a parent of the LysE variant of the present disclosure.

A sequence of the LysE of the present disclosure may be available in the NCBI GenBank, etc., which is a known database.

In one embodiment, the LysE of the present disclosure may be derived from the genus *Corynebacterium*, and may be derived from, for example, *Corynebacterium glutamicum*, *Corynebacterium suranareeae*, *Corynebacterium callunae*, *Corynebacterium deserti*, *Corynebacterium crudilactis*, *Corynebacterium efficiens*, *Corynebacterium pacaense*, but is not limited thereto.

In one embodiment, the LysE of the present disclosure may be a polypeptide of SEQ ID NO: 1. In one embodiment, the LysE of the present disclosure may be a polypeptide having about 60% or more sequence identity to the polypeptide of SEQ ID NO: 1, and any polypeptide may be included in the LysE protein without limitation, as long as it has activity identical or corresponding to that of the polypeptide consisting of the amino acid sequence of SEQ ID NO: 1.

For example, the LysE may be a protein derived from the genus *Corynebacterium* having at least about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 99.1%, or about 99.5% or more identity to SEQ ID NO: 1. Examples thereof may include proteins of NCBI Accession Nos. WP_003854734.1, P94633.2, WP_040967332.1, WP_074494034.1, WP_038583742.1, WP_074506107.1, WP_015439467.1, WP_220313759.1, WP_077311917.1, WP_172768514.1, WP_096455559.1, WP_015651045.1, WP_053544699.1, WP_066565316.1, NLZ57487.1, Q8RQM4.2, BAC18167.1, WP_006769321.1, WP_191733757.1, WP_080795286.1, WP_197088449.1, WP_047252903.1, WP_221709738.1, WP_123048290.1, WP_042621270.1, etc., but is not limited thereto.

In any one embodiment of the above-described embodiments, the LysE of the present disclosure may have or include the amino acid sequence of SEQ ID NO: 1, or may essentially consist of the amino acid sequence, but it may include polypeptides/proteins having the same activity without limitation. Further, it is apparent that any polypeptide/protein having an amino acid sequence having deletion, modification, substitution, or addition in part of the sequence may also be included within the scope of the polypeptide/protein to be mutated of the present disclosure as long as it has such homology or identity and exhibits L-lysine exporting activity.

As used herein, the term 'homology' or 'identity' refers to the degree of similarity between two given amino acid sequences or nucleotide sequences and may be expressed as a percentage. The terms 'homology and identity' may be often used interchangeably.

The sequence homology or identity of conserved polynucleotides or polypeptides may be determined by a standard alignment algorithm, and default gap penalties established by a program to be used may be used together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.](1988) SIAM J Applied Math 48: 1073). For example, homology, similarity, or identity may be determined using BLAST or ClustalW of the National Center for Biotechnology Information.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

As used herein, the "variant" refers to a polypeptide which has an amino acid sequence different from that of the variant before modification by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased as compared to that of the polypeptide before being varied. Further, some variants may include variants in which one or more portions such as an N-terminal leader sequence or a transmembrane domain have been removed. Other variants may include variants in which a portion of the N-and/or C-terminus has been removed from the mature protein. The term "variant" may be used interchangeably with terms such as modification, modified polypeptide, modified protein, mutant, mutein, and divergent and is not limited thereto as long as it is a term used with the meaning of variation.

Further, the variant may include deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be identified, purified, or synthesized.

The LysE variant provided in the present disclosure may be a polypeptide including an amino acid sequence in which any one or more amino acids of amino acids corresponding to positions 50, 153, and 215 of SEQ ID NO: 1 are substituted with another amino acid.

For example, the LysE variant of the present disclosure may be a variant including a substitution of another amino acid for any one or more amino acids of amino acids corresponding to positions 50, 153, and 215 from the N-terminus of SEQ ID NO: 1 in any polypeptide or protein having the lysine exporting activity.

The "another amino acid" is not limited as long as it is an amino acid other than the amino acid before substitution. Meanwhile, in the present disclosure, when expressed as 'a specific amino acid is substituted', it is obvious that the amino acid is substituted with an amino acid that is different from the amino acid before substitution, unless otherwise described as being substituted with a different amino acid.

In one embodiment, with regard to the variant of the present disclosure, in the amino acid sequence of SEQ ID NO: 1 which is a reference protein, the amino acid corresponding to position 50 may be an amino acid other than phenylalanine, and/or the amino acid corresponding to position 153 may be an amino acid other than asparagine, and/or the amino acid corresponding to position 215 may be an amino acid other than asparagine.

In any one embodiment of the above-described embodiments, with regard to the variant, the amino acid corresponding to position 50 of SEQ ID NO: 1 may be selected from glycine, alanine, valine, leucine, isoleucine, methionine, tryptophan, proline, serine, threonine, cysteine, tyrosine, asparagine, glutamine, aspartic acid, glutamic acid, lysine, histidine, and arginine. For example, the amino acid corresponding to position 50 of SEQ ID NO: 1 of the variant may be a nonpolar amino acid selected from glycine, alanine, valine, leucine, isoleucine, methionine, tryptophan, and proline. For example, the amino acid corresponding to position 50 of SEQ ID NO: 1 of the variant may be a branched amino acid selected from valine, leucine, and isoleucine, but is not limited thereto.

In any one embodiment of the above-described embodiments, with regard to the variant, the amino acid corresponding to position 153 of SEQ ID NO: 1 may be selected from glycine, alanine, valine, leucine, isoleucine, methionine, phenylalanine, tryptophan, proline, serine, threonine, cysteine, tyrosine, glutamine, aspartic acid, glutamic acid, lysine, histidine, and arginine. For example, the amino acid corresponding to position 153 of SEQ ID NO: 1 of the variant may be a polar amino acid selected from serine, threonine, cysteine, tyrosine, and glutamine. For example, the amino acid corresponding to position 153 of SEQ ID NO: 1 of the variant may be serine, threonine, or glutamine, but is not limited thereto.

In any one embodiment of the above-described embodiments, with regard to the variant, the amino acid corresponding to position 215 of SEQ ID NO: 1 may be selected from glycine, alanine, valine, leucine, isoleucine, methionine, phenylalanine, tryptophan, proline, serine, threonine, cysteine, tyrosine, glutamine, aspartic acid, glutamic acid, lysine, histidine, and arginine. For example, the amino acid corresponding to position 215 of SEQ ID NO: 1 of the variant may be a polar amino acid selected from serine, threonine, cysteine, tyrosine, and glutamine. For example, the amino acid corresponding to position 153 of SEQ ID NO: 1 of the variant may be serine, threonine, or glutamine, but is not limited thereto.

In any one embodiment of the above-described embodiments, the variant of the present disclosure may have substitution of any one or more amino acids of the amino acid residues at positions 50, 153, and 215 in the amino acid sequence of SEQ ID NO: 1 with another amino acid. In any one embodiment of the above-described embodiments, the variant of the present disclosure may have substitution of any one amino acid of the amino acid residues at positions 50, 153, and 215 in the amino acid sequence of SEQ ID NO: 1 with another amino acid. The amino acids after substitution of the amino acid residue at position 50, 153, or 215 in the amino acid sequence of SEQ ID NO: 1 may be the same as the amino acid after substitution of the amino acid residue corresponding to position 50, 153, or 215 in the amino acid sequence of SEQ ID NO: 1.

In any one embodiment of the above-described embodiments, the variant of the present disclosure may have substitution of any one or more amino acids of the amino acid residues at positions 50, 153, and 215 in the amino acid sequence of SEQ ID NO: 1 with another amino acid, and may have at least 60%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or more, or less than 100% identity to SEQ ID NO: 1.

In any one embodiment of the above-described embodiments, the variant of the present disclosure may consist of an amino acid sequence selected from SEQ ID NOS: 27 to 35, or may have or include an amino acid sequence having at least 60%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.5%, 99.8% or more, or 100% homology or identity thereto, or may essentially consist of the amino acid sequence, but is not limited thereto.

It is also apparent that a variant having an amino acid sequence having deletion, modification, substitution, conservative substitution, or addition of some sequence also falls within the scope of the present disclosure as long as the amino acid sequence has such a homology or identity and exhibits efficacy corresponding to that of the variant of the present disclosure.

Examples thereof include those having sequence addition or deletion that does not alter the function of the variant of the present disclosure at the N-terminus, C-terminus of the amino acid sequence, and/or inside the amino acid sequence, naturally occurring mutation, silent mutation, or conservative substitution.

The "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of proteins or polypeptides.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the numerical position of the amino acid residue corresponding to the amino acid residue of SEQ ID NO: 1. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto, and a sequence alignment program, a pairwise sequence comparison algorithm, etc., which known in the art, may be appropriately used.

Another aspect of the present disclosure provides a polynucleotide encoding the variant of the present disclosure.

As used herein, the term "polynucleotide" is a DNA or RNA strand having a certain length or more as a polymer of nucleotides in which nucleotide monomers are connected in a long chain by covalent bonds.

In the polynucleotide of the present disclosure, various modifications may be made in the coding region as long as the amino acid sequence is not changed in consideration of codon degeneracy or codons preferred in organisms that are intended to express the protein.

In any one embodiment of the above-described embodiments, the polynucleotide of the present disclosure may be available from the lysE sequence in the NCBI GenBank, etc., which is a known database.

Specifically, the polynucleotide encoding the LysE protein variant of the present disclosure may have or include a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, or may consist of or essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, but is not limited thereto. In the regard, in the sequence having the homology or identity, a codon encoding the amino acid corresponding to position 50 of SEQ ID NO: 1 may be one of codons encoding amino acids other than phenylalanine, and/or a codon encoding the amino acid corresponding to position 153 may be one of codons encoding amino acids other than asparagine, and/or a codon encoding the amino acid corresponding to position 215 may be one of codons (e.g., SEQ ID NO: 36 to 38) encoding amino acids other than asparagine, but is not limited thereto.

Further, the polynucleotide of the present disclosure may include a probe that may be prepared from a known gene sequence, for example, a sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al.,Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). Examples thereof include a condition in which polynucleotides having higher homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1X SSC, 0.1% SDS, specifically, 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

Still another aspect of the present disclosure provides a vector including the polynucleotide encoding the LysE variant. The LysE variant and the polynucleotide encoding the same are as described above.

In one embodiment, the vector may be an expression vector for expressing the polynucleotide in a host cell, but is not limited thereto.

The "vector" of the present disclosure may include a DNA construct including a polynucleotide sequence encoding a polypeptide of interest which is operably linked to a suitable expression regulatory region (or expression control sequence) so that the polypeptide of interest may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. The polynucleotide includes DNA and/or RNA encoding a polypeptide of interest. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the variant of the present disclosure.

Still another aspect of the present disclosure provides a microorganism including the LysE variant or the polynucleotide encoding the variant. The LysE variant and the polynucleotide encoding the same are as descried above.

The microorganism of the present disclosure may include any one or more of the LysE of the present disclosure variant, the polynucleotide encoding the same, and the vector including the polynucleotide.

As used herein, the term "microorganism (or strain)" includes all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of a polypeptide, protein, or product of interest.

The microorganism of the present disclosure may be a microorganism including any one or more of the variant of the present disclosure, the polynucleotide encoding the variant of the present disclosure, and a vector including the polynucleotide encoding the variant of the present disclosure; a microorganism modified to express the variant of the present disclosure or the polynucleotide encoding the variant of the present disclosure; a microorganism (e.g., a recombinant strain) expressing the variant of the present disclosure or the polynucleotide encoding the variant of the present disclosure; or a microorganism (e.g., a recombinant strain) having the activity of the variant of the present disclosure, but is not limited thereto.

The microorganism of the present disclosure may have L-arginine-exporting and/or producing ability.

The microorganism of the present disclosure may be a microorganism naturally expressing LysE protein or having arginine-exporting and/or producing ability, or a microorganism in which the variant of the present disclosure or the polynucleotide encoding the variant (or a vector including the polynucleotide) is introduced into a parent strain that does not have the LysE protein or arginine-exporting and/or producing ability, and/or in which arginine-exporting and/or producing ability is conferred on the parent strain, but is not limited thereto. For example, the microorganism of the present disclosure may be a microorganism in which the LysE of the present disclosure variant is introduced, and thus L-arginine-exporting and/or producing ability is increased, but is not limited thereto.

For example, the strain of the present disclosure is a cell or microorganism that is transformed with the polynucleotide of the present disclosure or a vector including the polynucleotide encoding the variant of the present disclosure to express the variant of the present disclosure. With respect to the objects of the present disclosure, the strain of the present disclosure may include any microorganism that includes the variant of the present disclosure and is able to export or produce L-arginine. For example, the strain of the present disclosure may be a recombinant strain in which the polynucleotide encoding the variant of the present disclosure is introduced into a natural wild-type microorganism or an L-arginine-producing microorganism to express the LysE variant and to have an increased L-arginine exporting and/or producing ability. The recombinant strain having the increased L-arginine exporting and/or producing ability may be a microorganism having an increased L-arginine exporting and/or producing ability, as compared to a natural wild-type microorganism or a microorganism in which LysE is unmodified (i.e., a microorganism expressing the wild-type LysE (SEQ ID NO: 1); or a microorganism that does not express the modified LysE protein), but is not limited thereto.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may be a strain into which the LysE variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

In one embodiment, the microorganism of the present disclosure may be a microorganism of the genus *Corynebacterium.* For example, the microorganism of the present disclosure may be *Corynebacterium glutamicum*, *Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, or *Corynebacterium flavescens*, specifically, *Corynebacterium glutamicum*, but is not limited thereto.

The microorganism exporting and/or producing L-arginine of the present disclosure may be characterized in that it includes the LysE variant disclosed in this specification, thereby having the enhanced L-arginine-producing and/or exporting ability as desired.

In any one embodiment of the above-described embodiments, the microorganism producing and/or exporting L-arginine or the microorganism having the L-arginine-producing and/or exporting ability in the present disclosure may be a microorganism in which some of the genes involved in the L-arginine biosynthesis pathway are enhanced or diminished, or some of the genes involved in the L-arginine degradation pathway are enhanced or diminished.

In any one embodiment of the above-described embodiments, the microorganism may be a microorganism in which some of the genes involved in the L-arginine biosynthesis pathway are enhanced or diminished. Examples of the L-arginine biosynthetic enzymes include N-acetylglutamyl phosphate reductase (argC), ornithine acetyl transferase (argJ), N-acetylglutamate kinase (argB), acetylornithine transaminase (argD), ornithine carbamoyl transferase (argF), argininosuccinic acid synthetase (argG), argininosuccinic acid lyase (argH), and carbamoyl phosphate synthetase (carAB). The arginine biosynthetic enzymes present on Arg operon (argCJBDFRGH) may be regulated by an arginine repressor encoded by argR (J Bacteriol. 2002Dec;184(23):6602-14.). Therefore, the L-arginine-producing ability may be provided or enhanced by deleting or diminishing the arginine repressor (argR) (US2002-0045223) and/or overexpressing one or more genes of the biosynthesis-related genes. For example, the arginine repressor may be a polypeptide represented by an amino acid sequence of SEQ ID NO: 39, but is not limited thereto.

As used herein, the term "diminishment" of the activity of a polypeptide has a concept encompassing all of the reduction of activity or the absence of activity, as compared to the intrinsic activity. The diminishment may be used interchangeably with inactivation, deficiency, down-regulation, decrease, reduction, attenuation, or the like.

The diminishment may also include a case where the activity of the polypeptide itself is reduced or eliminated compared to the activity of the polypeptide possessed by the original microorganism due to the mutation, etc. of the polynucleotide encoding the polypeptide, a case where the overall activity and/or concentration (expression level) of the polypeptides in the cell is lower compared to the native strain due to the inhibition of the expression of a gene of a polynucleotide encoding the polypeptide or the inhibition of translation into the polypeptide, a case where the expression of the polynucleotide is not made, and/or a case where the polypeptide has no activity in spite of the expression of the polynucleotide. The "intrinsic activity" refers to the activity of a specific polypeptide originally possessed by a parent strain before modification or a wild-type or unmodified microorganism, when transformed by genetic mutation caused by natural or artificial factors. This term may be used interchangeably with the "activity before modification". The "inactivation, deficiency, decrease, down-regulation, reduction, or attenuation" of the activity of a polypeptide compared to the intrinsic activity thereof means that the activity of the polypeptide is lowered compared to the activity of a specific polypeptide originally possessed by a parent strain before transformation or an unmodified microorganism.

The diminishment of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the diminishment of the polypeptide of the present disclosure may be achieved by:
1) deletion of the entirety or a part of the gene encoding the polypeptide;
2) modification of an expression control region (or expression control sequence) to reduce the expression of a gene encoding the polypeptide;
3) modification of the amino acid sequence constituting the polypeptide to eliminate or diminish the activity of the polypeptide (e.g., deletion/substitution/addition of one or more amino acids in the amino acid sequence);
4) modification of the gene sequence encoding the polypeptide to eliminate or diminish the activity of the polypeptide (e.g., deletion/substitution/addition of one or more nucleotides in the nucleotide sequence of the polypeptide gene to encode the polypeptide which is modified to eliminate or diminish the activity of the polypeptide);
5) modification of a nucleotide sequence encoding an initiation codon or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) combination of two or more selected from 1) to 8), but is not particularly limited thereto.

For example,
1) the deletion of a part or the entirety of the gene encoding the polypeptide may be the elimination of the entirety of the polynucleotide encoding an endogenous target protein in the chromosome, the replacement with a polynucleotide having deletion of some nucleotides, or the replacement with a marker gene.

Further, 2) the modification of an expression control region (or expression control sequence) may be the mutation on the expression control region (or expression control sequence) through deletion, insertion, non-conservative or conservative substitution, or a combination thereof, or the replacement with a sequence having more diminished activity. The expression control region includes a promoter, an operator sequence, a sequence for encoding a ribosomal binding site, and a sequence for controlling the termination of transcription and translation, but is not limited thereto.

Further, 3) and 4) the modification of the amino acid sequence or the polynucleotide sequence may be the mutation on the sequence through deletion, insertion, non-conservative or conservative substitution, or a combination thereof in the amino acid sequence of the polypeptide or the polynucleotide sequence encoding the polypeptide, or a replacement with an amino acid sequence or polynucleotide sequence modified to have more diminished activity or an amino acid sequence or polynucleotide sequence improved to have no activity, so as to diminish activity of the polypeptide, but is not limited thereto. For example, the expression of the gene may be inhibited or attenuated by introducing mutation into the polynucleotide sequence to form a termination codon, but is not limited thereto.

Further, 5) the modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a lower expression rate of the polypeptide, but is not limited thereto.

6) The introduction of an antisense oligonucleotide (e.g., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].

7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.

8) The addition of a promoter, which is to be reverse-transcribed, to the 3' end of the open reading frame (ORF) of the gene sequence encoding the polypeptide (reverse transcription engineering, RTE) may be making an antisense nucleotide complementary to the gene transcript encoding the polypeptide to diminish the activity of the polypeptide.

As used herein, the term "enhancement" of polypeptide activity means that the activity of a polypeptide is increased, as compared to the endogenous activity. The enhancement may be used interchangeably with terms such as activation, up-regulation, overexpression, and increase, etc. Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by the parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of the polypeptide is improved as compared to the activity and/or concentration (expression level) of a specific polypeptide originally possessed by the parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the corresponding polypeptide or in the amount of the product produced from the corresponding polypeptide.

For the enhancement of the activity of the polypeptide, various methods well known in the art may be applied, and the method is not limited as long as the activity of the polypeptide of interest may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the polypeptide;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically:
1) The increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing a vector capable of inserting the polynucleotide into a chromosome of a host cell into the host cell, but is not limited thereto. The vector is as described above.
2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may include, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.
   Examples of known strong promoters include CJ1 to CJ7 promoters (US Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. US 10584338 B2), O2 promoter (US Patent No. US 10273491 B2), tkt promoter, yccA promoter, etc., but is not limited thereto.
3) The modification of a nucleotide sequence encoding an initiation codon or 5'-UTR region of the gene transcript encoding the polypeptide may be, for example, substitution with a base sequence encoding another start codon having a higher polypeptide expression rate as compared to an endogenous start codon, but is not limited thereto.
4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion. The selection marker is as described above.
6) The introduction of a foreign polynucleotide exhibiting the activity of the polypeptide may be introduction of a foreign polynucleotide encoding a polypeptide exhibiting activity identical or similar to that of the polypeptide into a host cell. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.
7) The codon optimization of the polynucleotide encoding the polypeptide may be codon optimization of an endogenous polynucleotide so as to increase transcription or translation in a host cell, or codon optimization of a foreign polynucleotide so as to perform optimized transcription and translation in a host cell.
8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type microbial strain or a microbial strain before being modified, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may include a method using a DNA recombination technology. For example, by introducing a nucleotide sequence or vector including a nucleotide sequence homologous to the gene of interest into the microorganism to cause homologous recombination, a part or the entirety of the gene may be deleted. The nucleotide sequence or vector to be introduced may include a dominant selection marker, but is not limited thereto.

Still another aspect of the present disclosure provides a method of producing L-arginine, the method including the step of culturing the microorganism including the LysE variant or the polynucleotide encoding the same.

The LysE variant, polynucleotide encoding the same, microorganism including the same, and L-arginine are as described above.

The method of producing L-arginine of the present disclosure may include the step of culturing, in a medium, the microorganism including the LysE variant described herein or the polynucleotide encoding the same, or the vector including the polynucleotide.

As used herein, the term "culturing" means growing the microorganism of the present disclosure under appropriately controlled environmental conditions. The culturing process of the present disclosure may be performed according to suitable medium and culture conditions known in the art. Such a culture process may be easily adjusted and used by those skilled in the art according to the selected strain. Specifically, the culturing may be a batch type, continuous type, and/or fed-batch type, but is not limited thereto.

As used herein, the term "medium" means a mixed substance containing nutrients required to culture the microorganism of the present disclosure as a main component, and the medium supplies nutrients, growth factors, etc., including water, which are indispensable for survival and development. Specifically, as the medium and other culture conditions used for culturing the microorganism of the present disclosure, any one may be used without particular limitation as long as it is a medium used for common culture of microorganisms. The microorganism of the present disclosure may be cultured in a common medium containing proper carbon sources, nitrogen sources, phosphorus sources, inorganic compounds, amino acids and/or vitamins, etc., while controlling the temperature, pH, etc. under aerobic conditions.

For example, the culture medium for the strain of the genus *Corynebacterium* may be found in the document ["Manual of Methods for General Bacteriology" by the American Society for Bacteriology (Washington D.C., USA, 1981)].

In the present disclosure, the carbon sources include carbohydrates such as glucose, saccharose, lactose, fructose, sucrose, maltose, etc.; sugar alcohols such as mannitol, sorbitol, etc., organic acids such as pyruvic acid, lactic acid, citric acid, etc.; amino acids such as glutamic acid, methionine, lysine, etc. Further, natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (i.e., molasses converted to reducing sugar) may be used, and appropriate amounts of other carbon sources may be used in various manners without limitation. These carbon sources may be used singly or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, etc.; and organic nitrogen sources such as amino acids such as glutamic acid, methionine, glutamine, etc., peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, etc. may be used. In addition, amino acids, vitamins and/or suitable precursors, etc. may be included. These components or precursors may be added to the medium batchwise or continuously, but is not limited thereto.

Further, during the culturing of the microorganism of the present disclosure, the pH of the medium may be adjusted by adding compounds such as ammonium hydroxide, potassium hydroxide, ammonia, phosphoric acid, sulfuric acid, etc. to the medium in a proper manner. Further, during the culturing, foaming may be suppressed by using an antifoaming agent such as fatty acid polyglycol ester. Further, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but is not limited thereto.

In the culturing of the present disclosure, the culture temperature may be maintained at 20°C to 45°C, specifically, at 25°C to 40°C, and the strain may be cultured for about 10 to 160 hours, but are not limited thereto.

The L-arginine produced through the culturing of the present disclosure may be secreted into the medium or may remain in the cells.

The method of producing L-arginine of the present disclosure may further include the steps of preparing the microorganism of the present disclosure, preparing a medium for culturing the microorganism, or a combination thereof (in any order), for example, prior to the culturing step.

The method of producing L-arginine of the present disclosure may further include the step of recovering L-arginine from the medium according to the culturing (the medium subjected to the culturing) or from the microorganism. The recovering step may be further included after the culturing step.

The recovering may be to collect L-arginine of interest by way of a suitable method known in the art according to the method of culturing the microorganism of the present disclosure, for example, a batch, continuous, or fed-batch culture method. For example, centrifugation, filtration, treatment with a crystallized protein precipitant (salting out), extraction, sonication, ultrafiltration, dialysis, various forms of chromatography such as molecular sieve chromatography (gel filtration), adsorption chromatography, ion-exchange chromatography, and affinity chromatography, etc., HPLC, or a combination thereof may be used. L-arginine of interest may be recovered from the medium or microorganism by way of a suitable method known in the art.

The method of producing L-arginine of the present disclosure may further include a purification step. The purification may be performed by way of a suitable method known in the art. In an example, when the method of producing L-arginine of the present disclosure includes both the recovery step and the purification step, the recovery step and the purification step may be performed continuously or discontinuously regardless of the order, or may be performed simultaneously or by being combined into one step, but is not limited thereto.

In the method of the present disclosure, the LysE variant, polynucleotide, vector, microorganism/strain, etc. are as described in other aspects.

Still another aspect of the present disclosure provides a composition for producing L-arginine, the composition including the LysE variant of the present disclosure, the polynucleotide encoding the variant, the vector including the polynucleotide, or the microorganism including the polynucleotide of the present disclosure; a culture medium thereof; or a combination thereof.

The composition of the present disclosure may further include arbitrary suitable excipients which are commonly used in compositions for producing L-arginine. Such excipients may be, for example, a preservative, a wetting agent, a dispersing agent, a suspending agent, a buffering agent, a stabilizer, or an isotonic agent, etc., but are not limited thereto.

With regard to the composition of the present disclosure, the LysE variant, polynucleotide, vector, microorganism/strain, medium, etc. are as described in other aspects.

Still another aspect of the present disclosure provides a method of increasing an L-arginine-exporting and/or producing ability of a microorganism or a method of providing the L-arginine-exporting and/or producing ability for the microorganism, the method including the step of modifying the microorganism to express the LysE variant of the present disclosure.

With regard to the method of increasing an L-arginine-producing ability of a microorganism of the present disclosure, the variant, polynucleotide, vector, microorganism/strain, etc. are as described in other aspects.

The method of modifying the microorganism may include the step of introducing, into the microorganism, the LysE variant, the polynucleotide encoding the same, or the recombinant vector including the polynucleotide. However, the modification method is not limited thereto, and methods known in the art, including the method disclosed herein, may be used without limitation.

Still another aspect of the present disclosure provides use of the LysE variant of the present disclosure in exporting L-arginine.

Still another aspect of the present disclosure provides use of the microorganism including one or more of the LysE variant of the present disclosure, the polynucleotide encoding the variant, and the vector including the polynucleotide in producing L-arginine.

The LysE variant, polynucleotide, vector, microorganism are as described in other aspects.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Acquisition of various variants having substitution of amino acid residues at positions 50, 153, and 215 of LysE

The present inventors determined that amino acid residues at positions 50, 153, and 215 of SEQ ID NO: 1 are important positions for LysE activity, and prepared variants in which the corresponding amino acid residues were substituted with another amino acid. Specifically, genomic DNA of *Corynebacterium glutamicum* ATCC13869 was used as a template to perform PCR using primer pairs of Table 1 (SEQ ID NOS: 3 and 5, SEQ ID NOS: 4 and 6, SEQ ID NOS: 3 and 7, SEQ ID NOS: 4 and 8, SEQ ID NOS: 3 and 9, SEQ ID NOS: 4 and 10, SEQ ID NOS: 3 and 11, SEQ ID NOS: 4 and 12, SEQ ID NOS: 3 and 13, SEQ ID NOS: 4 and 14, SEQ ID NOS: 3 and 15, SEQ ID NOS: 4 and 16, SEQ ID NOS: 3 and 17, SEQ ID NOS: 4 and 18, SEQ ID NOS: 3 and 19, SEQ ID NOS: 4 and 20, SEQ ID NOS: 3 and 21, SEQ ID NOS: 4 and 22) and overlapping PCR using a primer pair of SEQ ID NOS: 3 and 4, thereby obtaining homologous recombination fragments, each having LysE-F50L, F50I, F50V, N153S, N153T, N153Q, N215T, N215S, or N215Q mutant sequence. At this time, PCR reaction was performed by 30 cycles consisting of denaturation at 95°C for 30 seconds; annealing at 55°C for 30 seconds; and extension at 72°C for 1 minute. Next, pDCM2 (Korean Patent Publication No. 10-2020-0136813) vector, which is not replicable in *Corynebacterium glutamicum,* and each fragment amplified by PCR were treated with BamHI and Xbal which are restriction enzymes for chromosome introduction, and then ligated using DNA ligase. Thereafter, each product was transformed into *E*. *coli* DH5α and plated on an LB solid medium containing kanamycin (25 mg/l).

**[Table 1]**

| SEQ ID NO. | Name | DNA sequence (5'-3') |
|---|---|---|
| 3 | lysE-AF | tcggtacccggggatcc CGCTTCATCTTCCAAGC |
| 4 | lysE-BR | tgcaggtcgactctaga GAGAACTTGTTGCAGTC |
| 5 | lysE-F50L-AR | AAGGTGCCGGCGATgagCAAAAAGACGT CAGAAAT |
| 6 | lysE-F50L-BF | ctcATCGCCGGCACCTTGGGCGTTGATCT TTTGTC |
| 7 | lysE-F501-AR | AAGGTGCCGGCGATgatCAAAAAGACGTC AGAAAT |
| 8 | lysE-F501-BF | atcATCGCCGGCACCTTGGGCGTTGATCT TTTGTC |
| 9 | lysE-F50V-AR | AAGGTGCCGGCGATgacCAAAAAGACGTC AGAAAT |
| 10 | lysE-F50V-BF | gtcATCGCCGGCACCTTGGGCGTTGATCT TTTGTC |
| 11 | lysE-N153S-AR | CATTCGGgctCAACCAGGTCAGCACGATT GCCATC |
| 12 | lysE-N153S-BF | CTGGTTGagcCCGAATGCGTATTTGGACG CGTTTG |
| 13 | lysE-N153T-AR | CATTCGGggtCAACCAGGTCAGCACGATT GCCATC |
| 14 | lysE-N153T-BF | CTGGTTGaccCCGAATGCGTATTTGGACG CGTTTG |
| 15 | lysE-N153Q-AR | CATTCGGttgCAACCAGGTCAGCACGATTG CCATC |
| 16 | lysE-N153Q-BF | CTGGTTGcaaCCGAATGCGTATTTGGACG CGTTTG |
| 17 | lysE-N215T-AR | CGACGACggtGATCCAGCGCCACACCTTG GGGCTG |
| 18 | lysE-N215T-BF | CTGGATCaccGTCGTCGTGGCAGTTGTGA TGACC |
| 19 | lysE-N215S-AR | CGACGACgctGATCCAGCGCCACACCTTG GGGCTG |
| 20 | lysE-N215S-BF | CTGGATCagcGTCGTCGTGGCAGTTGTGA TGACC |
| 21 | lysE-N215Q-AR | CGACGACttgGATCCAGCGCCACACCTTG GGGCTG |
| 22 | lysE-N215Q-BF | CTGGATCcaaGTCGTCGTGGCAGTTGTGA TGACC |

Colonies transformed with the plasmid into which the target gene was inserted were selected through PCR, and then each plasmid was obtained using a plasmid extraction method, and named pDCM2-lysE(F50L), pDCM2-lysE(F501), pDCM2-lysE(F50V), pDCM2-lysE(N153S), pDCM2-lysE(N153T), pDCM2-IysE(N153Q), pDCM2-lysE(N215T), pDCM2-lysE(N215S), pDCM2-lysE(N215Q), respectively.

### Example 2. Preparation of strain introduced with LysE variant derived from Corynebacterium glutamicum KCCM10741P and Evaluation of L-arginine producing ability

In order to introduce the LysE mutation into *Corynebacterium glutamicum* KCCM10741P (US 8034602 B2), which is an L-arginine producing strain, transformation was performed using the prepared recombinant plasmid (van der Rest et al., Appl Microbiol Biotechnol 52:541-545, 1999). Next, secondary recombination was performed on a solid plate medium containing 4% sucrose, and PCR was performed on the transformant in which secondary recombination was completed using a primer pair (SEQ ID NOS: 3 and 4) to identify the introduction of each mutation into LysE gene on the chromosome. At this time, PCR reaction was performed by 30 cycles consisting of denaturation at 95°C for 30 seconds; annealing at 55°C for 30 seconds; and extension at 72°C for 1 minute. The LysE variant-introduced strains selected therefrom were named KCCM10741P-lysE(F50L), KCCM10741P-lysE(F501), KCCM10741P-lysE(F50V), KCCM10741P-lysE(N153S), KCCM10741P-lysE(N153T), KCCM10741P-lysE(N153Q), KCCM10741P-lysE(N215T), KCCM10741P-lysE(N215S), KCCM10741P-lysE(N215Q), respectively.

To analyze the L-arginine producing ability of the LysE variant-introduced strains, they were cultured with the parent strain, *Corynebacterium glutamicum* KCCM10741P strain, in the following manner, respectively.

The parent strain, *Corynebacterium glutamicum* KCCM10741P, and the LysE variant strains prepared above were inoculated into a 250 ml corner-baffle flask containing 25 ml of a seed medium below, respectively, and cultured at 30°C with shaking at 200 rpm for 20 hours. Then, 1 ml of the seed culture was inoculated into a 250 ml corner-baffle flask containing 24 ml of a production medium and cultured at 30°C with shaking at 200 rpm for 72 hours. The compositions of the seed medium and the production medium are as follows.

### <Seed medium (pH 7.2)>

20 g of glucose, 45 g of ammonium sulfate, 2 g of magnesium sulfate heptahydrate, 2 g of potassium phosphate monobasic, 10 g of ammonium chloride, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate (based on 1 liter of distilled water)

### <Production medium (pH 7.2)>

60 g of glucose, 45 g of ammonium sulfate, 2 g of magnesium sulfate heptahydrate, 2 g of potassium phosphate monobasic, 10 g of ammonium chloride, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate, 30 g of calcium carbonate (based on 1 liter of distilled water)

After completion of the culture, the L-arginine producing ability was measured by HPLC (Waters 2478) (Table 2).

**[Table 2]**

| | Name of strain | L-arginine (g/L) | | | |
|---|---|---|---|---|---|
| | | Batch 1 | Batch 2 | Batch 3 | Mean |
| Control group | KCCM10741P | 3.1 | 2.9 | 2.9 | 3.0 |
| Experimental group | KCCM10741P-lysE(F50L) | 3.7 | 3.4 | 3.5 | 3.5 |
| | KCCM10741P-lysE(F50I) | 3.6 | 3.5 | 3.5 | 3.5 |
| | KCCM10741P-lysE(F50V) | 3,7 | 3.5 | 3.5 | 3.5 |
| | KCCM10741P-lysE(N153S) | 3.6 | 3.7 | 3.8 | 3.7 |
| | KCCM10741P-lysE(N153T) | 3.6 | 3.4 | 3.5 | 3.5 |
| | KCCM10741P-lysE(N153Q) | 3.5 | 3.7 | 3.5 | 3.6 |
| | KCCM10741P-lysE(N215T) | 3.6 | 3.8 | 3.6 | 3.7 |
| | KCCM10741P-lysE(N215S) | 3.5 | 3.5 | 3.7 | 3.6 |
| | KCCM10741P-lysE(N215Q) | 3.6 | 3.7 | 3.7 | 3.7 |

As a result, it was confirmed that all the LysE variant-introduced strains exhibited 20% increase in the L-arginine-producing ability, as compared to the parent strain.

### Example 3. Preparation of strain introduced with LysE variant derived from Corynebacterium glutamicum CJ1R having L-arginine producing ability and Evaluation of L-arginine producing ability

It was confirmed as follows whether or not other L-arginine-producing *Corynebacterium glutamicum* strains also had the same effect as in Example 2.

A *Corynebacterium glutamicum* strain having the L-arginine producing ability was prepared by introducing one kind of mutation (ΔargR) into a wild strain (ATCC13869). In detail, a recombinant vector for deletion of the gene argR (SEQ ID NO: 40) was constructed in the same manner as Example 1, and the primers used to construct the vector are shown in Table 3.

**[Table 3]**

| SEQ ID NO. | Name | DNA sequence (5'-3') |
|---|---|---|
| 23 | argR-AF | tcggtacccggggatccAGCAGGCCTTAAGGGTAAG |
| 24 | argR-AR | AGGGGCGCTGTCTTACCTCGGCTGGTGGGCCAGC |
| 25 | argR-BF | GGTAAGACAGCGCCCCTAGTTCAAGGCTTGTTAATC |
| 26 | argR-BR | tqcaggtcgactctagaACCGTTGAACTGCTTGCCAG |

The plasmid into which the desired fragment was inserted was selected through PCR, and this plasmid was named pDCM2-ΔargR. The wild-type *Corynebacterium glutamicum* strain was transformed using pDCM2-ΔargR in the same manner as Example 2, and the transformant was subjected to PCR using a primer pair (SEQ ID NOS: 23 and 26) to identify the strain with deletion of argR on the chromosome, which was named CJ1R.

Strain variants were prepared by introducing each of the nine LysE variants into CJ1R in the same manner as in example 2, and each was named CJ1R-lysE(F50L), CJ1R-lysE(F501), CJ1R-lysE(F50V), CJ1R-lysE(N153S), CJ1R-lysE(N153T), CJ1R-lysE(N153Q), CJ1R-lysE(N215T), CJ1R-lysE(N215S), CJ1R-lysE(N215Q).

To analyze the L-arginine producing ability of the CJ1R-derived LysE variant-introduced strains, the strains were cultured in the same manner as Example 2, and after completion of the culture, the L-arginine producing ability was measured by HPLC (Waters 2478) (Table 4).

**[Table 4]**

| | Name of strain | L-arginine (g/L) | | | |
|---|---|---|---|---|---|
| | | Batch 1 | Batch 2 | Batch 3 | Mean |
| Control group | CJ1R | 2 | 2.1 | 2.1 | 2.1 |
| Experimental group | CJ1R-lysE(F50L) | 2.5 | 2.4 | 2.5 | 2.5 |
| | CJ1R-lysE(F50I) | 2.4 | 2.6 | 2.5 | 2.5 |
| | CJ1R-lysE(F50V) | 2.6 | 2.5 | 2.6 | 2.6 |
| | CJ1R-lysE(N153S) | 2.4 | 2.4 | 2.6 | 2.5 |
| | CJ1R-lysE(N153T) | 2.6 | 2.7 | 2.4 | 2.6 |
| | CJ1R-lysE(N153Q) | 2.5 | 2.6 | 2.7 | 2.6 |
| | CJ1R-lysE(N215T) | 2.6 | 2.5 | 2.4 | 2.5 |
| | CJ1R-lysE(N215S) | 2.4 | 2.4 | 2.6 | 2.5 |
| | CJ1R-lysE(N215Q) | 2.6 | 2.5 | 2.7 | 2.6 |

As a result, it was confirmed that all the LysE variant-introduced strains exhibited 19% increase in the L-arginine-producing ability, as compared to the parent strain.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

**[Table 5]**

| | Sequence listing | |
|---|---|---|
| SEQ ID | Name | Sequence |
| NO. | | |
| 1 | LysE wt AA | MEIFITGLLLGASLLLSIGPQNVLVIKQGIKREGLIAVLLVCLISDVF LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLNPNAYLDAFVFIGGVGAQYGDTGRWIFA AGAFAASLIWFPLVGFGAAALSRPLSSPKVWRWINVVVAVVMTA LAIKLMLMG |
| 2 | LysE wt nt | ATGGAAATCTTCATTACAGGTCTGCTTTTGGGGGCCAGTCTT TTGCTGTCCATCGGACCGCAGAATGTACTGGTGATTAAACAA GGAATTAAGCGCGAAGGACTCATTGCGGTTCTTCTCGTGTGT TTAATTTCTGACGTCTTTTTGTTCATCGCCGGCACCTTGGGC GTTGATCTTTTGTCCAATGCCGCGCCGATCGTGCTCGATATTA TGCGCTGGGGTGGCATCGCTTACCTGTTATGGTTTGCCGTCA TGGCAGCGAAAGACGCCATGACAAACAAGGTGGAAGCGCC ACAGATCATTGAAGAAACAGAACCAACCGTGCCCGATGACA CGCCTTTGGGCGGTTCGGCGGTGGCCACTGACACGCGCAA CCGGGTGCGGGTGGAGGTGAGCGTCGATAAGCAGCGGGTT TGGGTGAAGCCCATGTTGATGGCAATCGTGCTGACCTGGTT GAACCCGAATGCGTATTTGGACGCGTTTGTGTTTATCGGCGG CGTCGGCGCGCAATACGGCGACACCGGACGGTGGATTTTC GCCGCTGGCGCGTTCGCGGCAAGCCTGATCTGGTTCCCGC TGGTGGGTTTCGGCGCAGCAGCATTGTCACGCCCGCTGTCC AGCCCCAAGGTGTGGCGCTGGATCAACGTCGTCGTGGCAG TTGTGATGACCGCATTGGCCATCAAACTGATGTTGATGGGTT AG |
| 3 | lysE-AF | tcggtacccggggatcc CGCTTCATCTTCCAAGC |
| 4 | lysE-BR | tgcaggtcgactctaga GAGAACTTGTTGCAGTC |
| 5 | lysE-F50L-AR | AAGGTGCCGGCGATgagCAAAAAGACGTCAGAAAT |
| 6 | lysE-F50L-BF | ctcATCGCCGGCACCTTGGGCGTTGATCTTTTGTC |
| 7 | lysE-F501-AR | AAGGTGCCGGCGATgatCAAAAAGACGTCAGAAAT |
| 8 | lysE-F501-BF | atcATCGCCGGCACCTTGGGCGTTGATCTTTTGTC |
| 9 | lysE-F50V-AR | AAGGTGCCGGCGATgacCAAAAAGACGTCAGAAAT |
| 10 | lysE-F50V-BF | gtcATCGCCGGCACCTTGGGCGTTGATCTTTTGTC |
| 11 | lysE-N153S-AR | CATTCGGgctCAACCAGGTCAGCACGATTGCCATC |
| 12 | lysE-N153S-BF | CTGGTTGagcCCGAATGCGTATTTGGACGCGTTTG |
| 13 | lysE-N153T-AR | CATTCGGggtCAACCAGGTCAGCACGATTGCCATC |
| 14 | lysE-N153T-BF | CTGGTTGaccCCGAATGCGTATTTGGACGCGTTTG |
| 15 | lysE-N153Q-AR | CATTCGGttqCAACCAGGTCAGCACGATTGCCATC |
| 16 | lysE-N153Q-BF | CTGGTTGcaaCCGAATGCGTATTTGGACGCGTTTG |
| 17 | lysE-N215T-AR | CGACGACggtGATCCAGCGCCACACCTTGGGGCTG |
| 18 | lysE-N215T-BF | CTGGATCaccGTCGTCGTGGCAGTTGTGATGACC |
| 19 | lysE-N215S-AR | CGACGACgctGATCCAGCGCCACACCTTGGGGCTG |
| 20 | lysE-N215S-BF | CTGGATCagcGTCGTCGTGGCAGTTGTGATGACC |
| 21 | lysE-N215Q-AR | CGACGACttgGATCCAGCGCCACACCTTGGGGCTG |
| 22 | lysE-N215Q-BF | CTGGATCcaaGTCGTCGTGGCAGTTGTGATGACC |
| 23 | argR-AF | tcggtacccggggatccAGCAGGCCTTAAGGGTAAG |
| 24 | argR-AR | AGGGGCGCTGTCTTACCTCGGCTGGTGGGCCAGC |
| 25 | argR-BF | GGTAAGACAGCGCCCCTAGTTCAAGGCTTGTTAATC |
| 26 | argR-BR | tgcaggtcgactctagaACCGTTGAACTGCTTGCCAG |
| 27 | LysE F50L AA | MEIFITGLLLGASLLLSIGPQNVLVIKQGIKREGLIAVLLVCLISDVF LLIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLNPNAYLDAFVFIGGVGAQYGDTGRWIFA AGAFAASLIWFPLVGFGAAALSRPLSSPKVWRWINVVVAVVMTA LAIKLMLMG |
| 28 | LysE N153S AA | MEIFITGLLLGASLLLSIGPQNVLVIKQGIKREGLIAVLLVCLISDVF LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLSPNAYLDAFVFIGGVGAQYGDTGRWIFA AGAFAASLIWFPLVGFGAAALSRPLSSPKVWRWINVVVAVVMTA LAIKLMLMG |
| 29 | LysE N215T AA | MEIFITGLLLGASLLLSIGPQNVLVIKQGIKREGLIAVLLVCLISDVF LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLNPNAYLDAFVFIGGVGAQYGDTGRWIFA AGAFAASLIWFPLVGFGAAALSRPLSSPKVWRWITVVVAVVMTA LAIKLMLMG |
| 30 | LysE F50I AA | MEIFITGLLLGASLLLSIGPQNVLVIKQGIKREGLIAVLLVCLISDVF LIIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLNPNAYLDAFVFIGGVGAQYGDTGRWIFA AGAFAASLIWFPLVGFGAAALSRPLSSPKVWRWINVVVAVVMTA LAIKLMLMG |
| 31 | LysE F50V AA | MEIFITGLLLGASLLLSIGPQNVLVIKQGIKREGLIAVLLVCLISDVF LVIAGTLGVDLLSNAAPIVLDIMRWGGIAYLLWFAVMAAKDAMT NKVEAPQIIEETEPTVPDDTPLGGSAVATDTRNRVRVEVSVDKQ RVWVKPMLMAIVLTWLNPNAYLDAFVFIGGVGAQYGDTGRWIF AAGAFAASLIWFPLVGFGAAALSRPLSSPKVWRWINVVVAVVM TALAIKLMLMG |
| 32 | LysE N153T AA | MEIFITGLLLGASLLLSIGPQNVLVIKQGIKREGLIAVLLVCLISDVF LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLTPNAYLDAFVFIGGVGAQYGDTGRWIFA AGAFAASLIWFPLVGFGAAALSRPLSSPKVWRWINVVVAVVMTA LAIKLMLMG |
| 33 | LysE N153Q AA | MEIFITGLLLGASLLLSIGPQNVLVIKQGIKREGLIAVLLVCLISDVF |
| | | LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLQPNAYLDAFVFIGGVGAQYGDTGRWIFA AGAFAASLIWFPLVGFGAAALSRPLSSPKVWRWINVVVAVVMTA LAIKLMLMG |
| 34 | LysE N215S AA | MEIFITGLLLGASLLLSIGPQNVLVIKQGIKREGLIAVLLVCLISDVF LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLNPNAYLDAFVFIGGVGAQYGDTGRWIFA AGAFAASLIWFPLVGFGAAALSRPLSSPKVWRWISVVVAVVMTA LAIKLMLMG |
| 35 | LysE N215Q AA | MEIFITGLLLGASLLLSIGPQNVLVIKQGIKREGLIAVLLVCLISDVF LFIAGTLGVDLLSNAAPIVLDIMRWGGIAYLLWFAVMAAKDAMTN KVEAPQIIEETEPTVPDDTPLGGSAVATDTRNRVRVEVSVDKQR VWVKPMLMAIVLTWLNPNAYLDAFVFIGGVGAQYGDTGRWIFA AGAFAASLIWFPLVGFGAAALSRPLSSPKVWRWIQVVVAVVMT ALAIKLMLMG |
| 36 | LysE F50L nt | ATGGAAATCTTCATTACAGGTCTGCTTTTGGGGGCCAGTCTT TTGCTGTCCATCGGACCGCAGAATGTACTGGTGATTAAACAA GGAATTAAGCGCGAAGGACTCATTGCGGTTCTTCTCGTGTGT TTAATTTCTGACGTCTTTTTGctcATCGCCGGCACCTTGGGCG TTGATCTTTTGTCCAATGCCGCGCCGATCGTGCTCGATATTAT GCGCTGGGGTGGCATCGCTTACCTGTTATGGTTTGCCGTCAT GGCAGCGAAAGACGCCATGACAAACAAGGTGGAAGCGCCA CAGATCATTGAAGAAACAGAACCAACCGTGCCCGATGACAC GCCTTTGGGCGGTTCGGCGGTGGCCACTGACACGCGCAAC CGGGTGCGGGTGGAGGTGAGCGTCGATAAGCAGCGGGTTT GGGTGAAGCCCATGTTGATGGCAATCGTGCTGACCTGGTTG AACCCGAATGCGTATTTGGACGCGTTTGTGTTTATCGGCGGC GTCGGCGCGCAATACGGCGACACCGGACGGTGGATTTTCG CCGCTGGCGCGTTCGCGGCAAGCCTGATCTGGTTCCCGCT GGTGGGTTTCGGCGCAGCAGCATTGTCACGCCCGCTGTCCA GCCCCAAGGTGTGGCGCTGGATCAACGTCGTCGTGGCAGTT GTGATGACCGCATTGGCCATCAAACTGATGTTGATGGGTTAG |
| 37 | LysE N153S nt | ATGGAAATCTTCATTACAGGTCTGCTTTTGGGGGCCAGTCTT TTGCTGTCCATCGGACCGCAGAATGTACTGGTGATTAAACAA GGAATTAAGCGCGAAGGACTCATTGCGGTTCTTCTCGTGTGT TTAATTTCTGACGTCTTTTTGTTCATCGCCGGCACCTTGGGC GTTGATCTTTTGTCCAATGCCGCGCCGATCGTGCTCGATATTA TGCGCTGGGGTGGCATCGCTTACCTGTTATGGTTTGCCGTCA TGGCAGCGAAAGACGCCATGACAAACAAGGTGGAAGCGCC ACAGATCATTGAAGAAACAGAACCAACCGTGCCCGATGACA CGCCTTTGGGCGGTTCGGCGGTGGCCACTGACACGCGCAA |
| | | CCGGGTGCGGGTGGAGGTGAGCGTCGATAAGCAGCGGGTT TGGGTGAAGCCCATGTTGATGGCAATCGTGCTGACCTGGTT GagcCCGAATGCGTATTTGGACGCGTTTGTGTTTATCGGCGG CGTCGGCGCGCAATACGGCGACACCGGACGGTGGATTTTC GCCGCTGGCGCGTTCGCGGCAAGCCTGATCTGGTTCCCGC TGGTGGGTTTCGGCGCAGCAGCATTGTCACGCCCGCTGTCC AGCCCCAAGGTGTGGCGCTGGATCAACGTCGTCGTGGCAG TTGTGATGACCGCATTGGCCATCAAACTGATGTTGATGGGTT AG |
| 38 | LysE N215T nt | ATGGAAATCTTCATTACAGGTCTGCTTTTGGGGGCCAGTCTT TTGCTGTCCATCGGACCGCAGAATGTACTGGTGATTAAACAA GGAATTAAGCGCGAAGGACTCATTGCGGTTCTTCTCGTGTGT TTAATTTCTGACGTCTTTTTGTTCATCGCCGGCACCTTGGGC GTTGATCTTTTGTCCAATGCCGCGCCGATCGTGCTCGATATTA TGCGCTGGGGTGGCATCGCTTACCTGTTATGGTTTGCCGTCA TGGCAGCGAAAGACGCCATGACAAACAAGGTGGAAGCGCC ACAGATCATTGAAGAAACAGAACCAACCGTGCCCGATGACA CGCCTTTGGGCGGTTCGGCGGTGGCCACTGACACGCGCAA CCGGGTGCGGGTGGAGGTGAGCGTCGATAAGCAGCGGGTT TGGGTGAAGCCCATGTTGATGGCAATCGTGCTGACCTGGTT GAACCCGAATGCGTATTTGGACGCGTTTGTGTTTATCGGCGG CGTCGGCGCGCAATACGGCGACACCGGACGGTGGATTTTC GCCGCTGGCGCGTTCGCGGCAAGCCTGATCTGGTTCCCGC TGGTGGGTTTCGGCGCAGCAGCATTGTCACGCCCGCTGTCC AGCCCCAAGGTGTGGCGCTGGATCaccGTCGTCGTGGCAGT TGTGATGACCGCATTGGCCATCAAACTGATGTTGATGGGTTA G |
| 39 | argR AA | MSLGSTPSTPENLNPVTRTARQALILQILDKQKVTSQVQLSELLL DEGIDITQATLSRDLDELGARKVRPDGGRAYYAVGPVDSIARED LRGPSEKLRRMLDELLVSTDHSGNIAMLRTPPGAAQYLASFIDR VGLKEVVGTIAGDDTVFVLARDPLTGKELGELLSGRTT |
| 40 | argR nt | ATGTCCCTTGGCTCAACCCCGTCAACACCGGAAAACTTAAAT CCCGTGACTCGCACTGCACGCCAAGCTCTCATTTTGCAGATT TTGGACAAACAAAAAGTCACCAGCCAGGTACAACTGTCTGAA TTGCTGCTGGATGAAGGCATCGATATCACCCAGGCCACCTTG TCCCGAGATCTCGATGAACTCGGTGCACGCAAGGTTCGCCC CGATGGGGGACGCGCCTACTACGCGGTCGGCCCAGTAGATA GCATCGCCCGCGAAGATCTCCGGGGTCCGTCGGAGAAGCT GCGCCGCATGCTTGATGAACTGCTGGTTTCTACAGATCATTC CGGCAACATCGCGATGCTGCGCACCCCGCCGGGAGCTGCC CAGTACCTGGCAAGTTTCATCGATAGGGTGGGGCTGAAAGA AGTCGTTGGCACCATCGCTGGCGATGACACCGTTTTCGTTCT CGCCCGTGATCCGCTCACAGGTAAAGAACTAGGTGAATTACT |
| | | CAGCGGGCGCACCACTTAA |

## Claims

1. A LysE variant, wherein any one or more amino acids of amino acids corresponding to positions 50, 153, and 215 of SEQ ID NO: 1 are substituted with another amino acid.

2. The LysE variant of claim 1, wherein the LysE variant is derived from the genus *Corynebacterium.*

3. The LysE variant of claim 1, wherein the amino acid corresponding to position 50 is substituted with a nonpolar amino acid.

4. The LysE variant of claim 1, wherein the amino acid corresponding to position 153 or 215 is substituted with a polar amino acid.

5. The LysE variant of claim 1, wherein any one or more amino acids of amino acid residues at positions 50, 153, and 215 in the amino acid sequence of SEQ ID NO: 1 are substituted with another amino acid.

6. The LysE variant of claim 1, wherein the LysE variant has 80% or more sequence identity to SEQ ID NO: 1.

7. A polynucleotide encoding the variant of any one of claims 1 to 6.

8. A microorganism of the genus *Corynebacterium,* the microorganism comprising a LysE variant, wherein any one or more amino acids of amino acids corresponding to positions 50, 153, and 215 of SEQ ID NO: 1 are substituted with another amino acid, or a polynucleotide encoding the LysE variant.

9. The microorganism of claim 8, wherein the microorganism is *Corynebacterium glutamicum.*

10. A method of producing L-arginine, the method comprising the step of culturing a microorganism of the genus *Corynebacterium,* the microorganism including a LysE variant, wherein any one or more amino acids of amino acids corresponding to positions 50, 153, and 215 of SEQ ID NO: 1 are substituted with another amino acid, or a polynucleotide encoding the LysE variant.
